# EUROPEAN PATENT APPLICATION

(11) **EP 2 000 124 A1**
(43) Date of publication of application: **10.12.2008**
(21) Application number: 08155669.8
(22) Date of filing: 06.05.2008
(51) Int. Cl.: A61K 8/41, A61Q 19/00

(54) **Cosmetic and pharmaceutical oil-in-water emulsions containing an ester quat**

(30) Priority: 08.06.2007 US 942711 P
(71) Applicant: Evonik Goldschmidt GmbH, 45127 Essen (DE)
(72) Inventor: Howe, Anna, Moseley, VA, 23120 (US); Dietz, Thomas, 63808, Haibach (DE); Grüning, Burghard, 45134, Essen (DE); Paez, Angela, Prince George, VA, 23875 (US)

(57) **Abstract**

The present invention relates to an ester quat of a defined structure useful for the preparation of cosmetic and pharmaceutical oil-in-water emulsions, and to oil-in-water emulsions which comprise said ester quat.

## Description

### Field of the Invention

The present invention relates to a cosmetic or pharmaceutical oil-in-water emulsion comprising an ester quat of a defined structure. The invention further relates the preparation of such an oil-in-water emulsion.

### Background of the Invention

Ester quats have been known as conditioning aids for hair and textile fiber in rinse off technologies. Ester quats in this context are generally understood to be quaternized fatty acid triethanolamine esters, which may be obtained by processes described in, e.g. WO 91/01295, wherein triethanolamine is partly esterified with fatty acids in the presence of hypophosphorous acid, air is passed through and the reaction product is quaternized with dimethyl sulfate or ethylene oxide. Triethanolamine based ester quats are typically complex mixtures composed of quat compounds having Formulas I, II and II below wherein each R independently denotes an alkyl group, which can be the same or different:

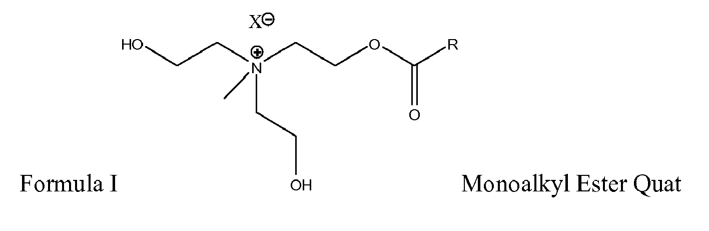

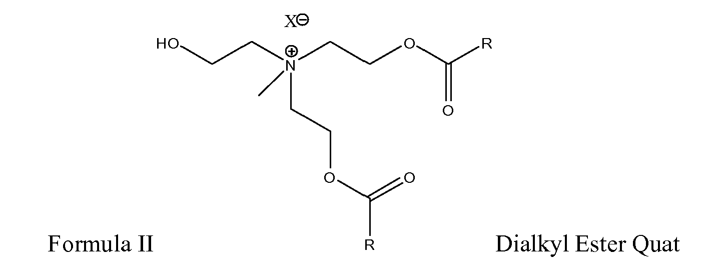

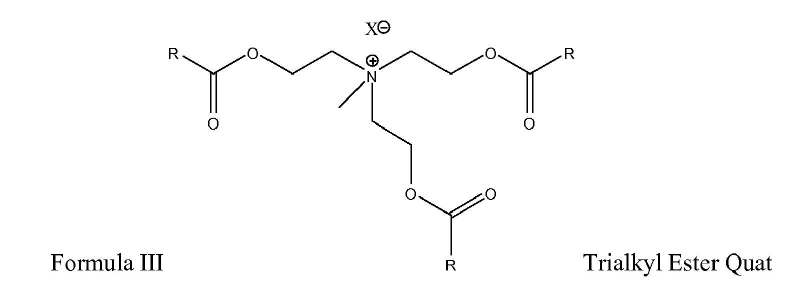

DE-C1 4308794 describes a process for the production of solid ester quats in which quaternization of triethanolamine esters is carried out in the presence of suitable dispersants, preferably fatty alcohols.

Overviews on this subject have been published, for example, by R. Puchta et al. in Tens. Surf. Det., 30, 186 (1993), by M. Brock in Tens. Surf, Det. 30, 394 (1993), by R. Lagerman et al. in J. Am. Oil. Chem. Soc., 71, 97 (1994) and by I. Shapiro in Cosm. Toil, 109, 77 (1994).

WO9101295, above, relates to the process for preparing quaternary ammonium compounds with an ester function which are used as textile softeners. Fatty acids are esterified with alkanolamine and the compounds so obtained are alkylated and can be used as readily biodegradable textile softeners.

EP0604726 reports a process for the quaternization of triethanolamine fatty acid esters and imidazoline amides with customary quaternizing agents in alkoxylated natural fats or oils or their mixtures with free fatty acids or mono- and/or diglycerides as the reaction medium, and to the use of the resulting reaction mixtures as laundry conditioner components.

### Summary of the Invention

The present invention is to a cosmetic or pharmaceutical emulsion comprising an ester quat of specific structure. The use of this particular quat provides superior stability and skin feel in oil-in-water (O/W) emulsions. In one practice, the ester quat of the invention is combined with one or more silicone compounds, which do not have emulsifying properties for O/W emulsions on their own, the result being a unique skin feel describable as "dry, yet caring," the combination further being made without compromising emulsion stability. Silicon compounds in this regard include, without limitation: organo-modified silicones; silicone quats; silicone polyols; polyglycerol-substituted silicones; silicone cross polymers.

In a first embodiment, the present invention is to a cosmetic or pharmaceutical emulsion comprising one or more of an ester quat of Formula IV below: 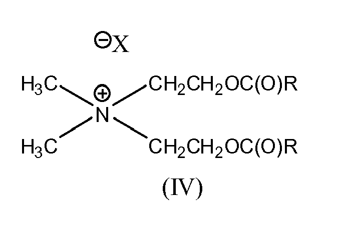 where
each R is independently an alkyl radical containing 15 to 21 carbon atoms in normal or branched configuration, wherein each R may independently optionally have an iodine number of 20 maximum, and
X is an anion selected from the group consisting of chloride, bromide, methosulfate, ethosulfate.

### Detailed Description of the Invention

The ester quat of Formula IV can be obtained by processes known in the art, e.g. by
(a) Esterifying a compound of Formula V 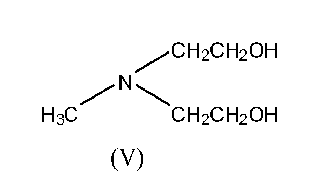 with an acid of the formula RCOOH, wherein R is as defined above, in the presence of acid having a pKa below about 5, under conditions effective to form a reaction product comprising a diester of Formula VI 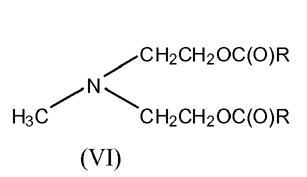
(b) reacting the diester of Formula (VI) with RX, where R is CH₃ and X is an anion as defined above. An example of an emulsifier of the present invention is diethanolamine distearic ester dimethyl ammonium chloride with the INCI name Distearoylethyl Dimonium Chloride.

The ester quat of Formula IV is useful, e.g., as an emulsifier for cosmetic or pharmaceutical oil-in-water emulsions with a skin friendly pH of 3.5 - 5.5, which emulsions are characterized by having excellent high-temperature and low-temperature stability, a brilliant appearance, and a pleasant feel on the skin, all as will be appreciated by those in the art. In addition, the emulsifier of the present invention include renewable raw materials, as opposed to oxidation-sensitive radicals, e.g., polyethylene glycol radical, and are thus biodegradable.

Using the ester quat of the present invention, it is possible to prepare an oil-in-water emulsion having a very fine degree of dispersion (which is synonymous with a brilliant appearance) and having excellent low-temperature and high-temperature stability, and whose pH can be adjusted to that of the natural pH of the skin. The emulsifier can be produced using vegetable raw materials. The inventive emulsifier is a highly effective oil-in-water emulsifier even in a very low concentration of < 1.6% by weight (e.g. Example 10), especially when it is combined with wax-like bodying agents ("polar waxes" or "hydrophilic waxes"), such as preferably glyceryl monodistearate, stearyl alcohol, cetyl alcohol or stearic acid.

In a further embodiment, the ester quat of the present invention is used in combination with one or more organo-modified silicones, which combination is especially utile for cosmetic or pharmaceutical oil-in-water emulsions with a skin friendly pH of 3.5 - 5.5, which combination is further characterized by having excellent high-temperature and low-temperature stability, a brilliant appearance, and a pleasant feel on the skin (e.g. Example 17,).

In another embodiment, the present invention is to the use of the ester quat of Formula IV in combination with one or more silicone quats for cosmetic or pharmaceutical oil-in-water emulsions with a skin friendly pH of 3.5 - 5.5 which are characterized by having excellent high-temperature and low-temperature stability, a brilliant appearance, and a pleasant feel on the skin (e.g. Example 6).

In another embodiment, the present invention is to the use of the ester quat of Formula IV in combination with one or more silicone copolyols for cosmetic or pharmaceutical oil-in-water emulsions with a skin friendly pH of 3.5 - 5.5, characterized by having excellent high-temperature and low-temperature stability, a brilliant appearance, and a pleasant feel on the skin (e.g. Example 16).

In another embodiment, the present invention is to the use of the ester quat of Formula IV in combination with one or more polyglycerol-substituted silicones for cosmetic or pharmaceutical oil-in-water emulsions with a skin friendly pH of 3.5-5.5, characterized by having excellent high-temperature and low-temperature stability, a brilliant appearance, and a pleasant feel on the skin (e.g. Example 11).

In another embodiment, present invention is to the use of the ester quat of Formula IV in combination with one or more silicone crosspolymers for cosmetic or pharmaceutical oil-in-water emulsions with a skin friendly pH of 3.5 - 5.5, characterized by having excellent high-temperature and low-temperature stability, a brilliant appearance, and a pleasant feel on the skin (e.g. Examples 19, 21).

The artisan will appreciate that the amounts for ester quat, and optionally the silicon compounds can vary consistent with the result being an emulsion. Without limitation, representatively, the ester quat may be present in up to about 10% wt., preferably about 2 to 8%; more preferably about 3 to about 6%; more still preferably about 4% to about 5%, e.g., about 4.5%. The silicon compound may be representatively present, from about 0.1% to about 5%; preferably about 0.5% to about 1%.

Among the emulsifying nonionic organo-modified silicones, those that are preferred have an HLB (Hydrophilic Lipophilic Balance) value ≤ 12; with HLB values < 10 being even more preferred.

In a particular embodiment, the present invention is directed to cosmetic or pharmaceutical oil-in-water emulsion comprising:
(a) at least one ester quat of Formula (IV);
(b) at least one liquid-crystalline-structure-forming hydrophilic wax, e.g., as a bodying agent and stabilizer;
(c) at least one cosmetic oil and/or wax; and
(d) at least one auxiliary and/or active ingredient as known in the art;
(e) optionally, at least one organo-modified silicone;
(f) optionally, at least one silicone crosspolymer; and
(g) optionally, at least one silicone quat.

Silicone quats which may be combined with the emulsifiers of the present invention are known from the state of the art. Without limitation, examples are given in the following documents: US 4,891,166, US 5,196,499, or US 5,098,079. Examples of commercially available silicone quats are ABIL® Quat 3272 (INCI: Quaternium-80), ABIL® Quat 3474 (INCI: Quaternium-80), or Silquat® AD (INCI: Silicone Quaternium-20).

The O/W emulsions of the invention may also contain additional emulsifiers such as , for example, non-ionic surfactants selected from at least one of the following groups:
- comb-like substituted nonionic silicones;
- Silicone copolyols
- Silicone crosspolymers
- addition products from 2 to 30 mol of ethylene oxide and/or 0 to 5 mol of propylene oxide to linear fatty alcohols having 8 to 22 carbon atoms, to fatty acids having 12 to 22 carbon atoms and to alkylphenols having 8 to 15 carbon atoms in the alkyl group;
- C₁₂ - C₁₈ fatty acid mono- and diesters of addition products of from 1 to 30 mol of ethylene oxide to glycerol;
- glycerol mono- and diesters and sorbitan mono- and diesters of saturated and unsaturated fatty acids having 6 to 22 carbon atoms and the ethylene oxide addition products thereof;
- alkyl mono- and oligoglycosides having 8 to 22 carbon atoms in the alkyl radical and the ethoxylated analogs thereof;
- addition products of from 15 to 60 mol of ethylene oxide with castor oil and/or hydrogenated castor oil;
- polyol and, in particular, polyglycerol esters, such as, for example, polyglycerol polyricinoleate, polyglycerol 12-hydroxystearate or polyglycerol dimerate. Also suitable are mixtures of compounds from two or more of these classes of substances;
- addition products of from 2 to 15 mol of ethylene oxide to castor oil and/or hydrogenated castor oil;
- partial esters based on linear, branched, unsaturated or saturated C₆ - C₂₂ fatty acids, ricinoleic acid and 12-hydroxystearic acid and glycerol, polyglycerol, pentaerythritol, dipentaerythritol, sugar alcohols (for example sorbitol), alkylglucosides (for example methylglucoside, butylglucoside, laurylglucoside), and polyglucosides (for example cellulose);
- mono-, di- and trialkyl phosphates, and mono-, di- and/or tri-PEG (Polyethylene Gycol) alkyl phosphates and salts thereof;
- wool wax alcohols;
- polysiloxane-polyalkyl-polyether copolymers, or corresponding derivatives;
- mixed esters of pentaerythritol, fatty acids, citric acid and fatty alcohol according to German patent DE 11 65 574 and/or mixed esters of fatty acids having 6 to 22 carbon atoms, methylglucose and polyols, preferably glycerol or polyglycerol:

- polyalkylene glycols;
- betaines;

The addition products of ethylene oxide and/or of propylene oxide to fatty alcohols, fatty acids, alkyl phenols, glycerol mono- and diesters and sorbitanmono- and diesters of fatty acids or to castor oil are known, commercially available products. These addition products are homolog mixtures, the average degree of alkoxylation of which corresponds to the ratio of the amounts of ethylene oxide and/or propylene oxide and substrate with which the addition reaction is carried out.

A further embodiment of the oil-in-water emulsions according to the present invention include suitable bodying agents which are also referred to as hydrophilic waxes. Suitable bodying agents are primarily fatty alcohols or hydroxyl fatty alcohols having 12 to 22, and preferably 16 to 18, carbon atoms, and also partial glycerides, fatty acids or hydroxy fatty acids. Examples are stearyl alcohol, stearic acid and glyceryl stearate.

Suitable thickeners are, for example, polysaccharides, in particular, agar, alginates and tyloses; carboxymethylcellulose and hydroxyethylcellulose. Also, higher molecular weight polyethylene glycol mono- and diesters of fatty acids,; polyacrylamides; polyvinyl alcohol; and polyvinylpyrrolidone, surfactants such as, for example, ethoxylated fatty acid glycerides, esters of fatty acids with polyols such as, for example, pentaerythritol or trimethylolpropane, fatty alcohol ethoxylates having a narrowed homolog distribution, or alkyl oligoglucosides may be employed herein.

Suitable as the oil phase are, for example, those oil components which are known as cosmetic and pharmaceutical oil components and as components of lubricants. These include, in particular, mono- or diesters of linear and/or branched mono- and/or dicarboxylic acids having 2 to 44 carbon atoms with linear and/or branched saturated or unsaturated alcohols having 1 to 22 carbon atoms. Also suitable within the meaning of the present invention are the esterification products of aliphatic difunctional alcohols having 2 to 36 carbon atoms with monofunctional aliphatic carboxylic acids having 1 to 22 carbon atoms. Monoesters suitable as oil components are, for example, the methyl esters and isopropyl esters of fatty acids having 12 to 22 carbon atoms, such as, for example, methyl laurate, methyl stearate, methyl oleate, methyl erucate, isopropyl palmitate, isopropyl - myristate, isopropyl stearate, and isopropyl oleate. Other suitable monoesters include, but are_not limited to: n-butyl stearate, n-hexyl laurate, n-decyl oleate, isooctyl stearate, isononyl palmitate, isononyl isononanoate, 2-ethylhexyl palmitate, 2-ethylhexyl laurate, 2-hexyldecyl stearate, 2-octyldodecyl palmitate, oleyl oleate, oleyl erucate, erucyl oleate, and esters obtainable from industrial aliphatic alcohol cuts and industrial, aliphatic carboxylic acid mixtures, for example, esters of unsaturated fatty alcohols having 12 to 22 carbon atoms and saturated and unsaturated fatty acids having 12 to 22 carbon atoms, as are accessible from animal and vegetable fats. Also suitable are naturally occurring monoesters or wax ester mixtures, as are present, for example, in jojoba oil or in sperm oil.

Suitable dicarboxylic esters include, but are not limited to: di-n-butyl adipate, di-n-butyl sebacate, di-(2-ethylhexyl) adipate, di-(2-hexyldecyl) succinate, D-isotridecyl acelate. Suitable diol esters are, for example, ethylene glycol dioleate, ethylene glycol diisotridecanoate, propylene glycol di-(2-ethyl hexanoate), butanediol diisostearate and neopentyl glycol dicaprylate.

Also suitable as an oil component are the fatty acid triglycerides, where, among these, the naturally occurring oils and fats are preferred. Suitable oil components include natural, vegetable oils, for example olive oil, sunflower oil, soy oil, peanut oil, rapeseed oil, almond oil, palm oil or else the liquid fraction of coconut oil or of palm kernel oil; and animal oils, such as, for example, neat's foot oil, the liquid fractions of beef tallow or also synthetic triglycerides of caprylic/capric acid mixtures, triglycerides of technical-grade oleic acid, of Isostearic acid or of palmitic acid/oleic acid mixtures. Also suitable as oil components are carbonates and ethers, for example diethylhexyl carbonate and didecyl carbonate or dioctylether.

Suitable further auxiliaries and additives are, inter alia, UV light protection filters which are well known to those skilled in the sunscreen art.

UV light protection filters are understood as meaning organic substances which are able to absorb ultraviolet rays and re-emit the absorbed energy in the form of long-wave radiation, for example heat. UVB filters may be oil-soluble. Examples of oil-soluble substances are:
- 3-benzylidenecamphor and derivatives thereof, for example 3-(4-methylbenzylidene)camphor;
- 4-aminobenzoic acid derivatives, preferably 2-ethylhexyl 4-(dimethylamino)benzoate, 2-ethylhexyl 4-(dimethylamino)benzoate and amyl 4-(dimethylamino)benzoate;
- esters of cinammic acid, preferably 2-ethylhexyl 4-methoxycinnamate, isopentyl 4-methoxycinnamate, 2-ethylhexyl 2-cyano-3-phenylcinnamate (octocrylene);
- esters of salicylic acid, preferably 2-ethylhexyl salicylate, 4-isopropylbenzyl salicylate, homomenthyl salicylate;
- derivatives of benzophenone, preferably 2-hydroxy-4-methoxybenzophenone, 2-hydroxy-4-methoxy-4'-methylbenzophenone, 2,2'-dihydroxy-4-methoxybenzophenone;
- esters of benzalmalonic acid, preferably di-2-ethylhexyl 4-methoxybenzalmalonate;
- triazine derivatives, such as[, for example,] 2,4,6-trianilino-(p-carbo-2'-ethyl-1'-hexyloxy)-1,3,5-triazine and octyltriazone; and
- propane-1,3-diones, such as 1-(4-tertbutylphenyl)-3-(4'-methoxyphenyl)propane-1,3-dione.

Suitable typical UV-A filters are, in particular, derivatives of benzoyl methane, such as 1-(4'-tert-butylphenyl) -3- (4'-methoxyphenyl)propane-1,3-dione or 1-phenyl-3-(4'-isopropylphenyl)propane-1,3-dione. The UV-A and UV-B filters can of course also be used in mixtures. In addition to said soluble substances, insoluble pigments, namely finely dispersed metal oxides or salts, are also suitable for this purpose, such as, for example, titanium dioxide, zinc oxide, iron oxide, aluminum oxide, cerium oxide, zirconium oxide, silicates (talc), barium sulfate and zinc stearate. Here, the particles of the UV filters should have an average diameter of less than 100 nm, preferably between 5 and 50 nm and in particular between 15 and 30 nm. The UV filters may have a spherical shape, although it is also possible to use particles which have an ellipsoidal shape or a shape which deviates in some other way from the spherical form. A relatively new class of light protection filters are micronized organic pigments, such as, for example, 2,2'-methylenebis-{6-(2H-benzotriazol-2-yl)-4-(1,1,3,3-tetramethylbutyl)phenol} having a particle size of less than 200 nm, which is available, for example, as a 50% strength aqueous dispersion.

In addition to the above-mentioned groups of primary light protection filters, it is also possible to use secondary light protection agents of the antioxidant type, which interrupt the photochemical reaction chain which is triggered when UV radiation penetrates into the skin. Typical examples thereof are amino acids (for example, glycine, histidine, tyrosine, tryptophan) and derivatives thereof, imidazole (for example, urocanic acid) and derivatives thereof, peptides such as D,L-carnosine, D-carnosine and derivatives thereof (for example anserine), carotinoids, carotenes (for example, α-carotene, β-carotene, lycopene) and derivatives thereof, chlorogenic acid and derivatives thereof, lipoic acid and derivatives thereof (for example, dihydrolipoic acid), aurothioglucose, propylthiouracil and other thiols (for example, thioredoxin, glutathione, cysteine, cystine, cystamine and the glycosyl, n-acetyl, methyl, ethyl, propyl, amyl, butyl and lauryl, palmitoyl, oleyl, γ-linoleyl, cholesteryl and glyceryl esters thereof) and salts thereof, dilauryl thiopropionate, distearyl thiopropionate, thiodipropionic acid and derivatives thereof (esters, ethers, peptides, lipids, nucleotides, nucleosides and salts) and sulfoximine compounds (for example, buthionine sulfoximines, homocysteine sulfoximine, buthionine sulfones, penta, hexa, heptathionine sulfoximine) in very low tolerated doses (for example, pmol to µmol/kg), and also (metal) chelating agents (for example, α-hydroxy fatty acids, palmitic acid, phytic acid, lactoferric acid), α-hydroxy acids (for example citric acid, lactic acid, malic acid), humic acid, bile acid, bile extracts, bilirubin, biliverdin, EDTA, EGTA and derivatives thereof, ubiquinone and ubiquinol and derivatives thereof, vitamin C and derivatives (e.g., ascorbyl palmitate, Mg ascorbyl phosphate, ascorbyl acetate), tocopherols and derivatives (for example, vitamin E acetate), vitamin A and derivatives (vitamin A palmitate), and coniferyl benzoate of benzoin resin, rutic acid and derivatives thereof, α-glycosylrutin, ferulic acid, furfurylideneglucitol, carnosine, butylhydroxytoluene, butylhydroxyanisole, nordihydroguaiacic acid, nordihydroguaiaretic acid, trihydroxybutyrophenone, uric acid and derivatives thereof, mannose and derivatives thereof, superoxide dismutase, zinc and derivatives thereof (for example, Zno, ZnSO₄), selenium and derivatives thereof (for example, selenomethionine), stilbenes and derivatives thereof (for example, stilbene oxide, trans-stilbene oxide) and the derivatives (salts, esters, ethers, sugars, nucleotides, peptides and lipids) of said active ingredients which are suitable according to the invention.

Suitable preservatives may also be employed in the present invention and include e.g., phenoxyethanol, formaldehyde solution, parabens, pentanediol or sorbic acid.

Suitable insect repellents may also be employed in the present invention and include, e.g. N,N-diethyl-m-toluamide, 1,2-pentanediol or Insect Repellent 3535, suitable self-tanning agents are dihydroxyacetone, and perfume oils which may be mentioned are mixtures of natural and synthetic fragrances. Natural fragrances are extracts from flowers (lily, lavender, rose, jasmine, neroli, ylang ylang), stems and leaves (geranium, patchouli, petitgrain), fruits (aniseed, coriander, caraway, juniper), fruit peels (bergamot, lemons, oranges), roots (mace, angelica, celery, cardamom, costus, iris, thyme), needles and branches (spruce, fir, pine, dwarf-pine), resins and balsams (galbanum, elemi, benzoin, myrrh, olibanum, opoponax). Also suitable are animal raw materials, such as, for example, civet and castoreum. Typical synthetic fragrance compounds are products of the ester, ether, aldehyde, ketone, alcohol and hydrocarbon type. Fragrance compounds of the ester type are e.g., benzyl acetate, phenoxyethyl isobutyrate, p-tert-butylcyclohexyl acetate, linalyl acetate, dimethylbenzylcarbinyl acetate, phenylethyl acetate, linalyl benzoate, benzyl formate, ethyl methylphenylglycidate, allyl cyclohexylpropionate, styrallyl - propionate and benzyl salicylate. The ethers include, for example, benzyl ethyl ether, the aldehydes include, for example, the linear alkanals having 8 to 18 carbon atoms, citral, citronellal, citronellyloxyacetaldehyde, cyclamenaldehyde, hydroxycitronellal, lilial and bourgeonal, the ketones include, for example, the ionones, α-isomethylionone and methyl cedryl ketone, the alcohols include anethole, citronellol, eugenol, isoeugenol, geraniol, linalool, phenylethyl alcohol and terpineol, and the hydrocarbons include predominately the terpenes and balsams. However, preference is given to using mixtures of different fragrances which together produce a pleasing scent note. Essential oils of relatively low volatility, which are mostly used as aroma components, are also suitable as perfume oils, for example, sage oil, camomile oil, oil of cloves, balm oil, mint oil, cinnamon leaf oil, lime blossom oil, juniperberry oil, vertiver oil, olibanum oil, galbanum oil, labolanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamenaldehyde, linalool, boisambrene forte, ambroxan, indole, Hedione, sandelice, lemon oil, mandarin oil, orange oil, allyl amyl glycolate, cyclovertal, lavandin oil, clary sage oil, β-damascone, geranium oil bourbon, cyclohexyl salicylate, Vertofix Coeur, Iso-E-Super, Fixolide NP, Evernyl, iraldein gamma, phenylacetic acid, geranyl acetate, benzyl acetate, rose oxide, Romillat, Irotyl and Floramat alone or in mixtures.

Suitable deodorant active ingredients may also be employed in the present invention and include e.g., odor-masking agents, such as the customary perfume constituents, odor absorbers, for example, the phyllosilicates described in laid-open patent specification DE-P 40 09 347, and of these, in particular, montmorillonite, kaolinite, illite, beidellite, nontronite, saponite, hectorite, bentonite, smectite, and also, for example, zinc salts of ricinoleic acid. Antibacterial agents are also suitable for incorporation into the oil-in-water emulsions according to the present invention. Advantageous substances are, for example, 2,4,4'-trichloro-2'-hydroxydiphenyl ether (Irgasan), 1,6-di(4-chlorophenylbiguanido)hexane (chlorhexidine), 3,4,4'-trichlorocarbanilide, quaternary ammonium compounds, oil of cloves, mint oil, thyme oil, triethyl citrate, farnesol (3,7,11-trimethyl-2,6,10-dodecatrien-1-ol) and the active agents described in patent laid-open specifications DE-198 55 934, DE-37 40 186, DE-39 38 140, DE-42 04 321, DE-42 29 707, DE-42 29 737, DE-42 38 081, DE-43 09 372, DE-43 24 219, EP 0666732, and DE 102004046603.

Customary antiperspirant active ingredients can likewise be advantageously used in the preparations according to the present invention, in particular astringents, e.g. basic aluminum chlorides, such as aluminum chlorohydrate ("ACH") and aluminum zirconium glycine salts ("ZAG").

Dyes may also be used where these substances are permitted and suitable for cosmetic purposes, as listed, e.g., in the publication "Kosmetische Färbemittel" from the Farbstoffkommission der Deutschen Forschungsgemeinschaft, Verlag Chemie, Weinheim, 1984, pp. 81-106. These dyes are customarily used in concentrations of from 0.001 to 0.1 % by weight, based on the total mixture.

Examples of suitable active ingredients are tocopherol, tocopherol acetate, tocopherol palmitate, ascorbic acid, deoxyribonucleic acid, retinol, bisabolol, allantoin, phytantriol, panthenol, AHA acids, amino acids, ceramides, pseudoceramides, essential oils, plant extracts and vitamin complexes.

The following examples are given to illustrate the present invention as well as to show certain advantages that may be obtained there from.

### EXAMPLES

Examples of oil-in-water skin care emulsions according to the present invention are listed below:

All formulas were made in 1000ml quantities. All formulation were homogenized for 4 minutes at high speed using a Greerco. Preservative Z is Germaben II.

### Processing

1. Mix Phase A and B separately, heating both up to 80°C.
2. Add Phase A to Phase B with stirring.
3. Homogenize.
4. Cool.
5. Add Phase C if present
6. Add Phase Z
7. Adjust pH to 4.2-4.3

### Example 1 (comparative):

| | | **WT%** |
|---|---|---|
| A | Diesterquat (Formula II, R = C₁₇H₃₅, X = CH3OSO3⁻) | 3.00 |
| | Petrolatum | 4.55 |
| | Ethylhexyl Palmitate | 4.25 |
| | Cetearyl Alcohol | 5.50 |
| B | Water | q.s to 100% |
| | NaC1 | 0.05 |
| | Glycerin | 7.65 |
| Z | Preservative | Q.S. |
| | Citric Acid | q.s. to 4.2-4.3 |

### Examples of oil-in-water skin care emulsions according to the present invention are listed below:

### Example 2:

| | | **WT %** |
|---|---|---|
| A | Diesterquat (Formula IV, R = C₁₇H₃₅, X = Cl⁻) | 3.00 |
| | Petrolatum | 4.55 |
| | Ethylhexyl Palmitate | 4.25 |
| | Cetearyl Alcohol | 5.50 |
| B | Water | q.s to 100% |
| | NaCl | 0.05 |
| | Glycerin | 7.65 |
| Z | Preservative | Q.S. |
| | Citric Acid | q.s. to 4.2-4.3 |

### Example 3:

| | | **WT%** |
|---|---|---|
| A | Diesterquat (Formula IV, R C₁₇H₃₃, X = C1⁻)¹⁾ | 3.00 |
| | Petrolatum | 4.55 |
| | Ethylhexyl Palmitate | 4.25 |
| | Cetearyl Alcohol | 5.50 |
| B | Water | q.s to 100% |
| | NaCl | 0.05 |
| | Glycerin | 7.65 |
| Z | Preservative | Q.S. |
| | Citric Acid | q.s. to 4.2-4.3 |

| | | |
|---|---|---|
| ¹⁾ R derived from Oleic Acid | | |

### Example 4:

| | | **WT %** |
|---|---|---|
| A | Diesterquat (Formula IV, R = C₁₇H₃₅, X = Cl⁻) | 3.25 |
| | Zinc Ricinoleate; Tetrahydroxypropyl Ethylenediamine; Laureth-3; Propylene Glycol | 2.00 |
| | Cetearyl Ethylhexanoate | 3.00 |
| | Cetearyl Alcohol | 1.75 |
| | Caprylic/Capric Triglyceride | 3.00 |
| | Dimethicone | 0.50 |
| B | Water | q.s to 100% |
| | Glycerin | 3.00 |
| Z | Preservative | Q.S. |
| | Citric Acid | q.s. to 6.0 |

### Example 5:

| | | **WT %** |
|---|---|---|
| A | Diesterquat (Formula IV, R = C₁₇H₃₅, X = Cl⁻) | 5.0 |
| | Petrolatum | 4.55 |
| | Ethylhexyl Palmitate | 4.25 |
| | Cetearyl Alcohol | 3.50 |
| | Dimethicone | 0.40 |
| B | Water | q.s to 100% |
| | NaC1 | 0.05 |
| | Glycerin | 7.65 |
| Z | Preservative | Q.S. |
| | Citric Acid | q.s. to 4.2-4.3 |

### Example 6:

| | | **WT %** |
|---|---|---|
| A | Diesterquat (Formula IV, R = C₁₇H₃₅, X = Cl⁻) | 6.40 |
| | Cetearyl Alcohol | 3.45 |
| | Isocetyl Palmitate | 6.00 |
| | Decyl Cocoate | 3.00 |
| | Cetyl Dimethicone | 0.75 |
| | C12-15 Alkyl Benzoate | 3.00 |
| B | Glycerin | 3.00 |
| | Water | 65.20 |
| | Creatine | 0.50 |
| C | Capryl/Capramidopropyl Betaine | 8.00 |
| Z | Phenonip | 0.70 |
| | Citric Acid (10% sol) for pH adjustment | q.s |

### Example 7:

| | | **WT %** |
|---|---|---|
| A | Diesterquat (Formula IV, R = C₁₇H₃₅, X Cl⁻) | 2.50 |
| | Quaternium-80¹⁾ | 2.38 |
| | Petrolatum | 4.55 |
| | Ethylhexyl Palmitate | 4.25 |
| | Cetearyl Alcohol | 3.62 |
| | Dimethicone | 0.40 |
| B | Water | 74.57 |
| | NaC1 | 0.08 |
| | Glycerin | 7.65 |
| C | Preservative | q.s. |
| | Citric Acid (10% sol) for pH adjustment | q.s |

| | | |
|---|---|---|
| ¹⁾ Degussa ABIL^{®} Quat 3474 | | |

### Example 8:

| | | **WT %** |
|---|---|---|
| A | Diesterquat (Formula IV, R = C₁₅H₃₁, X = CH₃SO₄⁻) | 4.50 |
| | Cetyl Dimethicone¹⁾ | 3.00 |
| | Caprylic/Capric Triglyceride | 4.00 |
| | C12-15 Alkyl Benzoate | 4.00 |
| | Cetearyl Alcohol | 2.00 |
| | Dimthicone | 0.50 |
| | Cyclopentasiloxane | 0.50 |
| | Mineral Oil | 4.00 |
| B | Glycerin | 3.00 |
| | Water | 74.50 |
| Z | Preservative, perfume | q. s. |
| | Citric Acid (10% sol) for pH adjustment | q.s |

| | | |
|---|---|---|
| ¹⁾Degussa ABIL^{®} Wax 9801 | | |

### Example 9:

| | | **WT%** |
|---|---|---|
| A | Diesterquat (Formula IV, R = C₁₇H₃₅, X = Cl⁻) | 3.00 |
| | Cetyl Dimethicone¹⁾ | 3.00 |
| | Diethylhexyl Carbonate | 7.00 |
| | C12-15 Alkyl Benzoate | 4.00 |
| | Cetearyl Alcohol | 2.50 |
| | Dimethicone | 0.50 |
| | Cyclopentasiloxane | 0.50 |
| | Mineral Oil | 4.00 |
| B | Glycerin | 3.00 |
| | Water | 72.50 |
| Z | Preservative, perfume | q. s. |
| | Citric Acid (10% sol) for pH adjustment | q.s |

| | | |
|---|---|---|
| ¹⁾Degussa ABIL^{®} Wax 9801 | | |

### Example 10:

| | | **WT %** |
|---|---|---|
| A | Diesterquat (Formula IV, R = C₁₇H₃₅, X = Cl⁻) | 4.00 |
| | Cetearyl Alcohol | 2.00 |
| | Diethylhexyl Carbonate | 2.00 |
| | Ethylhexyl Stearate | 3.00 |
| | Cyclopentasiloxane | 4.00 |
| B | Glycerin | 3.00 |
| | Water | 74.00 |
| | Propylene Glycol | 3.00 |
| C | Ceramide3, Ceramide6II, Ceramide I, Phytosphingosine, Chloresterol, Sodium Lauroyl Lactylate, Carbomer, Xanthan Gum | 5.00 |
| Z | Preservative, perfume | q. s. |
| | Citric Acid (10% sol) for pH adjustment | q.s |

### Example 11:

| | | **WT %** |
|---|---|---|
| A | Diesterquat (Formula IV, R = C₁₇H₃₅, X = Cl⁻) | 1.60 |
| | Polyglyceryl-3 Methylglucose Distearate | 2.00 |
| | Glyceryl Stearate SE | 1.00 |
| | Caprylic/Capric Triglyceride | 4.00 |
| | Stearyl Heptanoate | 2.00 |
| | Cetearyl Alcohol | 1.90 |
| | Triisostearin | 4.00 |
| | Tocoperyl Acetate | 0.45 |
| | Mineral Oil | 4.00 |
| B | Glycerin | 2.85 |
| | Water | 75.20 |
| | Creatine | 1.00 |
| Z | Preservative | q.s. |
| | Citric Acid (10% sol) for pH adjustment | q.s |

### Example 12:

| | | **WT %** |
|---|---|---|
| A | Diesterquat (Formula IV, R = C₁₇H₃₅, X = Cl⁻) | 5.85 |
| | Zinc Ricinoleate; Lysine; Propylene Glycol | 4.00 |
| | Cetearyl Alcohol | 3.15 |
| B | Glycerin | 3.00 |
| | Water | 81.00 |
| | Dipropylene Glycol | 3.00 |
| Z | Preservative | q.s. |
| | Citric Acid (10% sol) for pH adjustment | q.s |

### Example 13:

| | | **WT%** |
|---|---|---|
| A | Diesterquat (Formula IV, R = C₁₇H₃₅, X = Cl⁻) | 2.60 |
| | Cyclopentasiloxane | 15.00 |
| | Cetearyl Alcohol | 1.40 |
| B | Aluminium Chlorohydrate (50%) | 40.00 |
| | Propylene Glycol | 25.00 |
| | Water | 16.00 |
| Z | Preservative | q.s. |
| | Citric Acid (10% sol) for pH adjustment | q.s |

### Example 14:

| | | **WT %** |
|---|---|---|
| A | Diesterquat (Formula IV, R = C₁₇H₃₅, X = Cl⁻) | 3.90 |
| | Glyceryl Stearate | 1.00 |
| | PPG-15 Stearyl Ether | 4.70 |
| | Cetearyl Alcohol | 2.10 |
| B | Water | 26.3 |
| | Rezal 36G solution | 75.20 |
| | Creatine | 1.00 |
| Z | Preservative | q.s. |
| | Citric Acid (10% sol) for pH adjustment | q.s |

### Example 15:

| | | **WT %** |
|---|---|---|
| A | Diesterquat (Formula IV, R = C₁₇H₃₅, X = Cl⁻) | 3.00 |
| | Caprylic/Capric Triglyceride | 9.00 |
| | Cetearyl Ethylhexanoate | 9.00 |
| | Cetearyl Alcohol | 1.50 |
| B | Glycerin | 3.00 |
| | Water | 74.50 |
| Z | Preservative | q.s. |
| | Citric Acid (10% sol) for pH adjustment | q.s |

### Example 16:

| | | **WT %** |
|---|---|---|
| A | Diesterquat (Formula IV, R = C₁₇H₃₅, X = Cl⁻) | 2.50 |
| | Bis-PEG/PPG-16/16 PEG/PPG-16/6 Dimethicone; Caprylic/Capric Triglyceride¹⁾ | 1.00 |
| | Caprylic/Capric Triglyceride | 9.00 |
| | Cetearyl Ethylhexanoate | 9.00 |
| | Cetearyl Alcohol | 1.00 |
| B | Glycerin | 3.00 |
| | Water | 74.50 |
| Z | Preservative | q.s. |
| | Citric Acid (10% sol) for pH adjustment | q.s |

| | | |
|---|---|---|
| ¹⁾ Degussa ABIL^{®} Care 85 | | |

### Example 17:

| | | **WT %** |
|---|---|---|
| A | Diesterquat (Formula IV, R = C₁₇H₃₅, X = C1⁻) | 2.50 |
| | Cetyl PEG/PPG-10/1 Dimethicone¹⁾ | 1.00 |
| | Caprylic/Capric Triglyceride | 9.00 |
| | Cetearyl Ethylhexanoate | 9.00 |
| | Cetearyl Alcohol | 1.00 |
| B | Glycerin | 3.00 |
| | Water | 74.50 |
| Z | Preservative | q.s. |
| | Citric Acid (10% sol) for pH adjustment | q.s |

| | | |
|---|---|---|
| ¹⁾ Degussa ABIL^{®} EM 90 | | |

### Example 18:

| | | **WT %** |
|---|---|---|
| A | Diesterquat (Formula IV, R = C₁₇H₃₅, X = C1⁻) | 2.50 |
| | Cetyl PEG/PPG-10/1 Dimethicone¹⁾ | 0.50 |
| | Quaternium 80²⁾ | 0.50 |
| | Caprylic/Capric Triglyceride | 9.00 |
| | Cetearyl Ethylhexanoate | 9.00 |
| | Cetearyl Alcohol | 1.00 |
| B | Glycerin | 3.00 |
| | Water | 74.50 |
| Z | Preservative | q.s. |
| | Citric Acid (10% sol) for pH adjustment | q.s |

| | | |
|---|---|---|
| ¹⁾ Degussa ABIL^{®} EM 90 ²⁾ Degussa ABIL^{®} Quat 3474 | | |

### Example 19:

| | | **WT %** |
|---|---|---|
| A | Diesterquat (Formula IV, R = C₁₇H₃₅, X = Cl⁻) | 2.50 |
| | Lauryl Polyglyceryl-3 Polydimethylsiloxayethyl Dimethicone¹⁾ | 1.00 |
| | Caprylic/Capric Triglyceride | 9.00 |
| | Cetearyl Ethylhexanoate | 9.00 |
| | Cetearyl Alcohol | 1.00 |
| B | Glycerin | 3.00 |
| | Water | 74.50 |
| Z | Preservative | q.s. |
| | Citric Acid (10% sol) for pH adjustment | q.s |

| | | |
|---|---|---|
| ¹⁾ Shin-Etsu KF-6105 | | |

### Example 20:

| | | **WT %** |
|---|---|---|
| A | Diesterquat (Formula IV, R = C₁₇H₃₅, X = Cl⁻) | 2.50 |
| | Lauryl Polyglyceryl-3 Polydimethylsiloxayethyl Dimethicone¹⁾ | 0.50 |
| | Quaternium 80²⁾ | 0.50 |
| | Caprylic/Capric Triglyceride | 9.00 |
| | Cetearyl Ethylhexanoate | 9.00 |
| | Cetearyl Alcohol | 1.00 |
| B | Glycerin | 3.00 |
| | Water | 74.50 |
| Z | Preservative | q.s. |
| | Citric Acid (10% sol) for pH adjustment | q.s |

| | | |
|---|---|---|
| ¹⁾ Shin-Etsu KF-6105 ²⁾ Degussa ABIL^{®} Quat 3474 | | |

### Example 21:

| | | **WT %** |
|---|---|---|
| A | Diesterquat (Formula IV, R = C₁₇H₃₅, X = Cl⁻) | 2.50 |
| | Cyclopentasiloxane; Dimethicone/Vinyl Dimethicone Crosspolymer¹⁾ | 1.00 |
| | Caprylic/Capric Triglyceride | 9.00 |
| | Cetearyl Ethylhexanoate | 9.00 |
| | Cetearyl Alcohol | 1.00 |
| B | Glycerin | 3.00 |
| | Water | 74.50 |
| Z | Preservative | q.s. |
| | Citric Acid (10% sol) for pH adjustment | q.s |

| | | |
|---|---|---|
| ¹⁾ Shin-Etsu KSG-15 | | |

### Example 22:

| | | **WT %** |
|---|---|---|
| A | Diesterquat (Formula IV, R = C₁₇H₃₅, X = Cl⁻) | 2.50 |
| | Bis-PEG/PPG-16/16 PEG/PPG-16/6 Dimethicone; Caprylic/Capric Triglyceride¹⁾ | 0.50 |
| | Cyclopentasiloxane; Dimethicone/Vinyl Dimethicone Crosspolymer | 0.50 |
| | Caprylic/Capric Triglyceride | 9.00 |
| | Cetearyl Ethylhexanoate | 9.00 |
| | Cetearyl Alcohol | 1.00 |
| B | Glycerin | 3.00 |
| | Water | 74.50 |
| Z | Preservative | q.s. |
| | Citric Acid (10% sol) for pH adjustment | q.s |

| | | |
|---|---|---|
| ¹⁾ Degussa ABIL^{®} Care 85 | | |

A skin panel test was run to evaluate the sensory profile of selected formulations using the following criteria: - = bad, o = fair, + = good, ++ = very good

| | | **1** | **2** | **7** | **16** | **17** | **18** | **20** | **21** | **22** |
|---|---|---|---|---|---|---|---|---|---|---|
| A | Diesterquat (Formula IV, R = C₁₇H₃₅, X = Cl⁻) | | 3.00 | 2.50 | 2.50 | 2.50 | 2.50 | 2.50 | 2.50 | 2.50 |
| | Diesterquat(Formula II, R = C₁₇H₃₅, X = CH3OSO3⁻) | 3.00 | | | | | | | | |
| | Quaternium-80 | | | 2.38 | | | 0.50 | 0.50 | | |
| | Bis-PEG/PPG-16/16 PEG/PPG-16/6 Dimethicone; Caprylic/Capric Triglyceride | | | | 1.00 | | | | | 0.50 |
| | Cetyl PEG/PPG-10/1 Dimethicone | | | | | 1.00 | 0.50 | | | |
| | Cyclopentasiloxane; Dimethicone/Vinyl Dimethicone Crosspolymer | | | | | | | | 1.00 | 0.50 |
| | Lauryl Polyglyceryl-3 Polydimethylsiloxayethyl Dimethicone | | | | | | | 0.50 | | |
| | Caprylic/Capric Triglyceride | 9.00 | 9.00 | | 9.00 | 9.00 | 9.00 | 9.00 | 9.00 | 9.00 |
| | Petrolatum | | | 4.55 | | | | | | |
| | Cetearyl Ethylhexanoate | 9.00 | 9.00 | | 9.00 | 9.00 | 9.00 | 9.00 | 9.00 | 9.00 |
| | Ethylhexyl Palmitate | | | 4.55 | | | | | | |
| | Cetearyl Alcohol | 1.50 | 1.50 | 3.62 | 1.00 | 1.00 | 1.00 | 1.00 | 1.00 | 1.00 |
| | Dimethicone | | | 0.40 | | | | | | |
| B | Glycerin | 3.00 | 3.00 | 7.65 | 3.00 | 3.00 | 3.00 | 3.00 | 3.00 | 3.00 |
| | Water | 74.50 | 74.50 | 74.50 | 74.50 | 74.50 | 74.50 | 74.50 | 74.50 | 74.5 0 |
| | NaCl | | | 0.08 | | | | | | |
| Z | Preservative | q.s. | q.s. | | q.s. | q.s. | q.s. | q.s. | q.s. | q.s. |
| | Citric Acid (10% sol) for pH adjustment | q.s. | q.s. | | q.s. | q.s. | q.s. | q.s. | q.s. | q.s. |

The results are reported in Table 1.

**TABLE 1**

| Formulation | Skin feel |
|---|---|
| 1 (comparative) | o wet, breaks easily |
| 2 | + dry, caring |
| 7 | + caring rich |
| 16 | ++ silky, cooling |
| 17 | ++ caring, smooth, soft |
| 18 | ++ dry, smooth, soft |
| 20 | ++ caring, silky |
| 21 | ++ powdery, dry |
| 22 | ++ dry, silky |

All formulations were evaluated on the fore arm and the after feel was evaluated.

While the present invention has been particularly shown and described with respect to preferred embodiments thereof, it will be understood by those skilled in the art that the foregoing and other changes in forms and details may be made therein without departing from the spirit and scope of the present invention. It is therefore intended that the present invention not be limited to the exact forms and details but fall within the scope of the appended Claims.

## Claims

1. A cosmetic or pharmaceutical oil-in-water emulsion comprising the ester quat of Formula (IV) 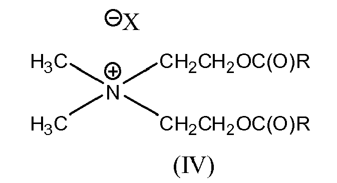 where
each R is independently an alkyl radical containing 15 to 21 carbon atoms in normal or branched configuration, wherein each R may independently optionally may have an iodine number of 20 max; and
X is an anion selected from the group consisting of chloride, bromide, methosulfate, ethosulfate.

2. The cosmetic or pharmaceutical oil-in-water emulsion of claim 1 further comprising mixtures of a liquid-crystalline-structure forming hydrophilic waxes; cosmetic waxes; cosmetic oils; customary auxiliaries; or active ingredients, and optionally additional coemulsifiers.

3. The cosmetic or pharmaceutical oil-in-water emulsion of claim 1 further comprising an organo-modified silicone.

4. The cosmetic or pharmaceutical oil-in-water emulsion of claim 1 further comprising a silicone quat.

5. The cosmetic or pharmaceutical oil-in-water emulsion of claim 1 further comprising of a silicone copolyol.

6. The cosmetic or pharmaceutical oil-in-water emulsion of claim 1 further comprising a polyglycerol substituted silicone derivative.

7. The cosmetic or pharmaceutical oil-in-water emulsion of claim 1 further comprising a silicone crosspolymer.

8. The cosmetic or pharmaceutical oil-in-water emulsion of Claim 3 wherein the further auxiliaries and additives are selected from the group consisting of UV light protection filters, antioxidants, preservatives, insect repellents, self-tanning agents, perfume oils, dyes and active ingredients.

9. The cosmetic or pharmaceutical oil-in-water emulsion of claim 1 further comprising of a silicone quat and a silicone copolyol.
